# EUROPÄISCHE PATENTSCHRIFT

(11) **EP 0 694 280 B1**
(45) Veröffentlichungstag und Bekanntmachung des Hinweises auf die Patenterteilung: **11.07.2001**
(21) Anmeldenummer: 95114514.3
(22) Anmeldetag: 22.10.1991
(51) Int. Cl.: A61B 1/00, A61B 17/34

(54) **Instrument zum Penetrieren von Körpergewebe**
Instrument for penetrating body tissue
Instrument pour pénétrer le tissu d'un corps

(30) Priorität: 06.11.1990 DE 4035146
(43) Veröffentlichungstag der Anmeldung: 31.01.1996
(62) Teilanmeldung aus: 91117958.8
(73) Patentinhaber: PARTOMED Medizintechnik GmbH, D-78667 Villingendorf (DE)
(72) Erfinder: Riek, Siegfried, Dr. med., D-78628 Rottweil (DE); Bachmann, Karl-Heinz, D-78667 Villingendorf (DE); Gaiselmann, Thomas, D-78667 Villingendorf (DE)
(74) Vertreter: Patentanwälte Westphal, Mussgnug & Partner

(56) Entgegenhaltungen:
- EP-A- 0 349 955
- DE-A- 1 616 107
- US-A- 3 357 433

## Beschreibung

Die Erfindung betrifft ein Instrument zum Penetrieren von Körpergewebe.

Instrumente dieser Gattung dienen insbesondere als Trokare dazu, einen künstlichen Zugang zu Körperhöhlen oder Organen zu schaffen, die keine natürliche Verbindung nach außen aufweisen. Das Instrument weist eine Spitze auf, die dazu dient, das Körpergewebe zu durchbohren und die Perforationsöffnung auf den Durchmesser des Schafts aufzuweiten. Bei Verwendung als Trokar wird dabei eine den Schaft umschließende Hülse zusammen mit dem Trokar in die Perforationsöffnung vorgeschoben und stellt nach dem Abziehen des Trokars den künstlichen Zugang zu der Körperhöhle dar, durch welchen Endoskope, Instrumente und dergleichen in das Körperinnere eingeführt werden können.

Das Einstechen des Trokars bringt auch bei günstiger Wahl des Einstichortes die Gefahr mit sich, daß Blutgefäße im Unterhautfettgewebe, in der Muskelfascie und im Peritoneum (Bauchfell), also Gefäße der Bauchdecke, verletzt werden. Weiter besteht nach dem Penetrieren der Bauchdecke die Gefahr, daß Gefäße des Abdominalraumes (Bauchhöhle) und Organe des Bauchraumes, wie Dickdarm, Dünndarm, Omentum Majus (großes Netz) und retroperitoneal liegende Gefäße und Strukturen verletzt werden können. Besonders verletzungsgefährdet ist der Dünndarm und das Omentum Majus, wenn Adhäsionen und Verwachsungen mit der vorderen Bauchwand bestehen, so daß beim Penetrieren der Bauchwand gleichzeitig die mit dieser verwachsenen Strukturen durchbohrt werden können, ehe der Trokar in die freie Bauchhöhle gelangt. Um die Verletzungsgefahr insbesondere des Darmes und des Omentum Majus zu reduzieren, kann zunächst unter Anheben der Bauchdecke eine Hohlnadel durch die Bauchdecke geführt werden, um Gas in die Bauchhöhle einzuleiten und die Bauchdecke von dem darunterliegenden Omentum Majus und Darm für das anschließende Einstechen des Trokars zu distanzieren. Auch hierbei bleibt jedoch beim Einstechen der Hohlnadel und des Trokars ein Restrisiko der Verletzung.

Ein aus DE 29 22 239 C2 bekanntes Instrument weist ein Außenrohr auf, das an seinem distalen vorderen Ende abgeschrägt ist, um eine Einstechspitze zu bilden. In dem Außenrohr sind zwei Lichtleiterbündel zur Spitze geführt, deren vordere Endflächen in der Ebene der abgeschrägten Stirnfläche des Außenrohres liegen. Das Licht einer Lichtquelle wird durch ein Lichtleiterbündel geführt und tritt an der distalen Spitze aus. Das zweite Lichtleiterbündel empfängt den reflektierten Anteil dieses austretenden Lichtes und leitet diesen zu einem lichtempfindlichen Element. Die gemessene Intensität des reflektierten Lichts stellt eine Information über die anatomische Struktur vor der Spitze dar. Die Änderung der Intensität des reflektierenden Lichts läßt erkennen, wenn sich die Spitze des Instruments im freien Bauchraum einem Organ nähert. Beim Durchdringen aufeinanderfolgender Gewebeschichten der Bauchdecke oder beim Eindringen der Spitze in durch Adhäsionen und Verwachsungen mit der Bauchwand verbundene Organe ändert sich jedoch das Reflexionsvermögen der Gewebestrukturen vor der Spitze praktisch nicht, so daß eine Kontrolle des Eindringens der Spitze nicht möglich ist. Die Abschrägung des Außenrohres ergibt eine Einstechspitze, die an dem Mantel des Außenrohres seitlich neben der Austrittsfläche der Lichtleiterbündel angeordnet ist und diese überragt. Die Einstechspitze beschränkt dadurch das über das optische System kontrollierbare Sichtfeld.

Aus DE-AS 16 16 107 ist ein als Mikroskop dienendes faseroptisches System bekannt, das in einer Einstechnadel angeordnet ist. Das das optische System umschließende Außenrohr der Nadel ist an dem distalen Ende abgeschrägt, um die Einstechspitze zu bilden. Über das optische System kann das Gewebe vor der Spitze der Nadel mikroskopisch beobachtet werden. Die Nadel ist nicht als Trokar verwendbar, da ihr geringer Durchmesser nicht zu einer Aufweitung des Perforationsloches führt. Die durch die Abschrägung des Außenrohres erzeugte Einstechspitze erzeugt einen das Sichtfeld einschränkenden toten Winkel.

Aus der EP 0 369 936 A1 und EP 0 347 140 A1 sind Endoskope bekannt, die ein Außenrohr aufweisen, in welchem faseroptische Systeme zur Spitze geführt sind. Diese Endoskope ermöglichen eine Beobachtung in Richtung der an der Spitze austretenden Lichtleiterfasern. Die Endoskope sind nicht gut zum Penetrieren von Gewebe geeignet.

Aus der EP 0 369 937 A1 ist ein solches Endoskop bekannt, welches die Merkmale des Oberbegriffs des Anspruchs 1 besitzt und insbesondere als Arthroskop verwendet wird. In dem Außenrohr sind koaxial eine Optik und eine diese umschließende, aus Lichtleitfasern gebildete Beleuchtungseinheit angeordnet. Die Optik ist bis an das distale Ende des Außenrohres geführt und bildet mit ihren Objektivlinsen das distale Ende des Endoskopes. Die die distale Objektivlinse umschließende Beleuchtungseinheit und das Außenrohr sind nach hinten konisch abgeschrägt. Das Arthroskop dient zur Beobachtung von Gelenkinnenräumen.

Aus DE-OS 22 18 901 ist ein massiver Trokar bekannt, dessen Trokarhülse an ihrem proximalen Ende eine äußere Schraubenwindung aufweist. Die Schraubenwindung dient dazu, die gesetzte Trokarhülse zuverlässiger gegen eine axiale Verschiebung in der durch den Trokar erzeugten Perforationsöffnung festzulegen.

Der Erfindung liegt die Aufgabe zugrunde, ein Instrument zum Penetrieren von Körpergewebe zur Verfügung zu stellen, das durch eine verbesserte optische Kontrolle beim Einstechen das Risiko von Verletzungen von Gefäßen, Organen und dergleichen weitestmöglich reduziert.

Diese Aufgabe wird bei einem gattungsgemäßen Instrument erfindungsgemäß durch die im kennzeichnenden Teil des Patentanspruchs 1 angegebenen Merkmale gelöst.

Eine vorteilhafte Ausführungsform der Erfindung ist im abhängigen Anspruch 2 angegeben.

Das erfindungsgemäße Instrument weist einen hohlen Schaft auf. Die Spitze oder zumindest der distale Endteil der Spitze ist als Fenster aus einem geeigneten durchsichtigen Material ausgebildet, z.B. aus Glas, Quarzglas, Plexiglas oder dergleichen. Durch den hohlen Schaft ist eine Optik zu der Spitze geführt, z.B. eine Optik mit Glasfaserlichtleiter, wie sie in Endoskopen verwendet wird. Weiter ist in dem hohlen Schaft eine Beleuchtungseinheit zur Spitze geführt. Die Beleuchtungseinheit kann in die Optik integriert werden, indem zur Beleuchtung dienende Lichtleiterfasern in den Strang der Optik eingesetzt sind. Ebenso ist es möglich, die Beleuchtung getrennt von der Optik durch den Schaft in die Spitze zu führen oder auch in die Optik integrierte Beleuchtungseinheiten mit zusätzlichen getrennt geführten Beleuchtungseinheiten zu kombinieren. Die Optik endet in einem axialen Abstand hinter dem Scheitelpunkt der Spitze, so daß durch die Optik die gesamte Mantelfläche des kegelförmigen Fensters beleuchtet und beobachtet werden kann. Der Operateur hat somit beim Vorschub des Instruments Sicht auf die vor der Spitze des Instruments liegenden Strukturen, die penetriert werden. Der Operateur kann also z.B. Blutgefäße erkennen, bevor'diese von der Spitze des Instruments getroffen werden, und diesen ausweichen. Insbesondere kann auch der wichtige Schritt des Durchdringens des Peritoneums unter Sicht erfolgen. Das semitransparene Peritoneum läßt bereits vor der vollständigen Durchdringung einen Einblick in den Abdominalraum zu, so daß das darunter liegende Omentum Majus, die Därme sowie Gefäßstrukturen des Peritoneums erkennbar sind und deren Verletzung vermieden werden kann. Außerdem kann der Operateur beim Vorschub des Instruments die seitlich an der Mantelfläche des Fensters vorbeiwandernden, von der Spitze durchdrungenen Strukturen beobachten und bekommt dadurch ein Gefühl für das Eindringen der Spitze und für die Vorschubgeschwindigkeit.

Das Fenster dient aufgrund seiner Kegelform als Spitze, die sowohl das Penetrieren des Gewebes als auch das Aufweiten des Perforationsloches bewirkt. Die spezielle Gestalt des kegelförmigen Fensters ist dabei von geringerer Bedeutung. Bevorzugt wird ein gerader Kreiskegel, da dieser am einfachsten herzustellen ist und die geringste optische Verzerrung bei der Beobachtung liefert. Grundsätzlich sind jedoch auch andere Kegelformen möglich, z.B. mit polygonaler Grundfläche und mit leicht balliger oder leicht eingezogener Mantellinie.

Da die Beleuchtungseinheit innerhalb des die Spitze bildenden Fensters angeordnet ist und die Beleuchtung durch dieses Fenster erfolgt, kann es vorteilhaft sein, die Innenfläche des Fensters zu entspiegeln. Die Kegelform des Fensters ergibt bei hohler Spitze jedoch zwangsläufig eine Schrägstellung der inneren Fensterflächen gegenüber der optischen Achse der Beleuchtung und der Optik, so daß störende Reflexionen ohnehin gering sind.

Vorteilhafterweise kann die Beobachtung des vor der Spitze des Instruments liegenden Bereiches noch zusätzlich verbessert werden, so daß sich für den Operateur eine Kombination des statischen Bildes des vor der Spitze des Instruments liegenden Bereichs mit dem dynamischen Bild des Vordringens der Spitze ergibt. In dieser Ausführung ist in die das Fenster bildende Spitze zusätzlich zu der die gesamte Mantelfläche des Fensters betrachtenden Optik eine zweite Optik geführt, die als dünne flexible Glasfaser-Optik ausgebildet ist. Diese zweite Optik ist seitlich an der ersten Optik vorbei in die das Fenster bildende Spitze geführt und endet in der Stirnfläche der das Fenster bildenden Spitze. Diese zweite Optik mit möglichst geringem Durchmesser ergibt somit die Sicht auf den Bereich vor der Spitze, während die erste Optik die Sicht auf die an der Mantelfläche des Fensters anliegenden durchdrungenen Strukturen ermöglicht. Der kleine Durchmesser der zweiten Faseroptik behindert die Beobachtung durch die erste Optik praktisch nicht.

In dieser Art kann der Operateur durch zwei Okulare sowohl das Vordringen der Spitze des Instruments beobachten, um ein Gefühl für Vorschubweg und Vorschubgeschwindigkeit zu bekommen, als auch die vor der Spitze liegenden Strukturen beobachten, um Verletzungen von Gefäßen, von Organen, des Darmes oder des Omentum Majus zu vermeiden.

In allen Fällen sind die Optiken vorzugsweise als Weitwinkeloptik (fish eye-Optik) ausgebildet, um dem Operateur ein möglichst großes Sichtfeld zu bieten und eine Beobachtung durch die gesamte Mantelfläche des kegelförmigen Fensters zu ermöglichen.

Ist das Instrument als Trokar mit einer Trokar-Hülse ausgebildet, so kann bei dem Einstechen des Trokars unter Sicht die Möglichkeit eines Ausweichens verletzungsgefährdeter Strukturen optimal ausgenützt werden, wenn die Hülse ein Außengewinde aufweist. Die Hülse ist axial unverschieblich jedoch drehbar auf dem Trokar gelagert. Durch Drehen der Hülse greift deren Außengewinde in das Gewebe und bewirkt den Vorschub des Trokars. Der Trokar mit seiner Optik dreht sich dabei nicht. Der Vorschub über die mit Gewinde versehene Hülse ermöglicht ein gleichmäßiges ruckfreies Eindringen der Trokarspitze, ohne daß der Operateur einen axialen Druck auf den Trokar ausüben muß. Dies begünstigt ein feinfühliges Führen der Trokarspitze im Bereich verletzungsgefährdeter Strukturen.

Um die Hülse ohne größeren Kraftaufwand drehen zu können, kann diese über eine Ratsche manuell angetrieben werden. Ebenso ist ein Antrieb über einen Elektromotor mit entsprechender Untersetzung möglich.

Das erfindungsgemäße Instrument wird vorzugsweise als Trokar zum Penetrieren der Bauchdecke und zum Einführen einer Trokar-Hülse in die Perforationsöffnung verwendet.

Im folgenden wird die Erfindung anhand von in der Zeichnung dargestellten Ausführungsbeispielen näher erläutert. Es zeigen :
- Figur 1: eine Teil- und Seitenansicht eines als Trokar ausgebildeten Insruments,
- Figur 2: einen Teil- und Axialschnitt der Spitze des Trokars,
- Figur 3: eine Stirnansicht der Spitze von hinten,
- Figur 4: eine Teil-und Seitenansicht des Trokars in einer bevorzugten Variante,
- Figur 5: einen Tell- und Axialschnitt der Spitze des Trokars der Figur 4,
- Figur 6: einen Figur 5 entsprechenden Axialschnitt einer abgewandelten Variante und
- Figur 7: eine Seitenansicht der Spitze des Trokars in der erfindungsgemäßen Ausführungsform.

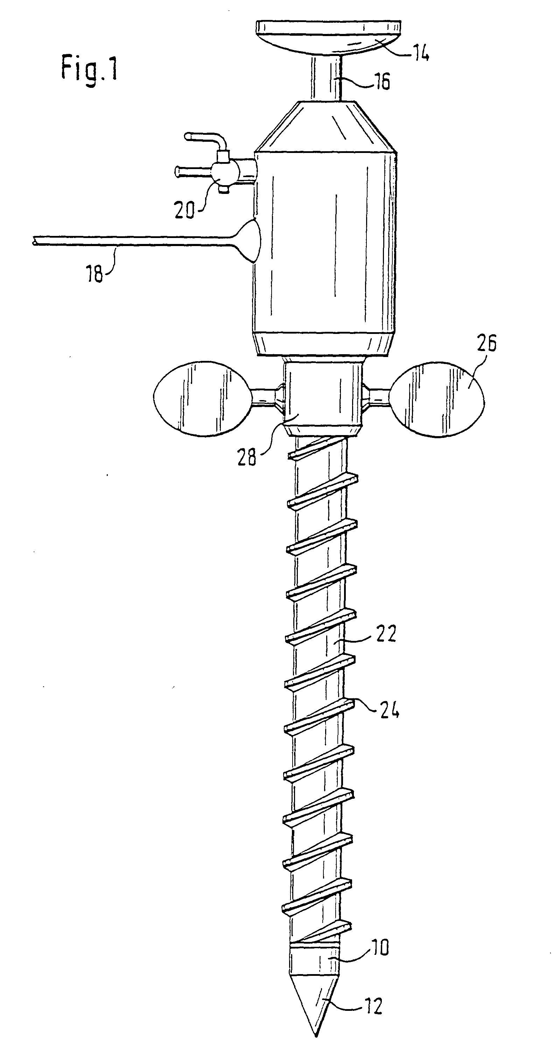

Der in Figur 1 dargestellte Trokar weist einen hohlen zylindrischen Schaft 10 aus Stahl auf, in dessen distales vorderes Ende eine nachfolgend beschriebene Spitze 12 eingesetzt ist. Am proximalen hinteren Ende des Schaftes 10 sitzt das mit einer Augenmuschel 14 versehene Okular 16 einer nachfolgend beschriebenen koaxial in dem Schaft 10 angeordneten Optik. Weiter ist seitlich in das hintere Ende des Schaftes 10 ein Lichtleiter 18 einer nachfolgend beschriebenen Beleuchtungseinheit eingeführt. Schließlich ist am hinteren Ende des Schaftes 10 ein Insufflationshahn 20 angeordnet, durch welchen in an sich bekannter Weise z.B. CO₂-Gas zu nicht dargestellten Austrittsöffnungen am vorderen distalen Schaftende zugeführt werden kann.

Auf dem Schaft 10 ist eine Hülse 22 drehbar aber axial unverschieblich gelagert. Die Hülse 22 weist an ihrem Außenmantel ein stumpfes Schraubengewinde 24 auf. Die Hülse 22 kann gegenüber dem Schaft 10 über eine mit Griffknebeln 26 versehene Ratsche 28 drehend angetrieben werden. Ebenso ist ein elektromotorischer Antrieb der Hülse 22 möglich. Wird die Hülse 22 bei eingestochenem Trokar gedreht, so bewirkt das Schraubengewinde 24 einen axialen Vorschub des Trokars, wobei dieser selbst sich nicht dreht. Mit Hilfe der mit dem Schraubengewinde 24 versehenen Hülse 22 kann der Trokar ohne Ausübung eines axialen Druckes durch den Operateur langsam und feinfühlig in dem Gewebe vorgeschoben werden.

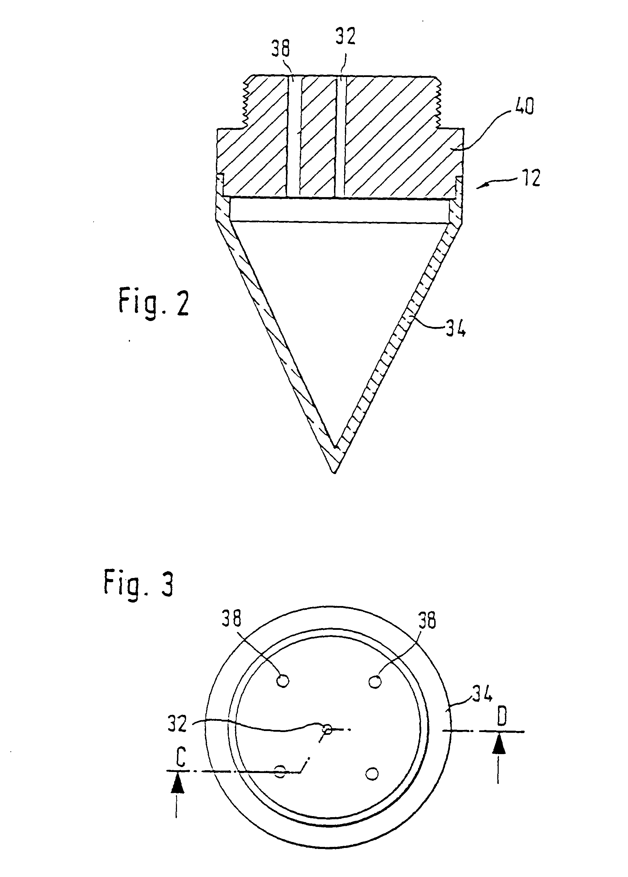

Wie aus den Figuren 2 und 3 zu ersehen ist, ist in das distale Ende des hohlen Schaftes 10 eine Spitze 12 eingesetzt, z.B. eingeschraubt oder eingelötet.

In eine axial mittig in die Spitze 12 führende Bohrung ist eine Optik 32 eingesetzt. Die Optik 32 ist axial durch den gesamten Schaft 10 hindurchgeführt und endet am proximalen hinteren Ende in dem Okular 16. Das distal vordere Ende der Optik 32 ist in die Bohrung der Spitze 12 eingekittet oder eingeklebt.

Die Optik 32 ist eine an sich bekannte Weitwinkel-Optik, wie sie beispielsweise für Endoskope verwendet wird. Vorzugsweise wird eine Lichtleiter-Optik verwendet.

Parallel zu der die Optik 32 aufnehmenden mittigen Bohrung sind vier weitere Bohrungen in der Spitze 12 vorgesehen, die in gleichem Abstand um die mittige Bohrung angeordnet sind. In diese Bohrungen ist jeweils eine Beleuchtungseinheit 38 in Form eines Lichtleiters eingesetzt. Die Lichtleiter der Beleuchtungseinheiten 38 sind durch den Schaft 10 geführt. Den Beleuchtungseinheiten 38 wird über den Lichtleiter 18 und eine Verzweigung Licht zugeführt.

Die Spitze 12 besteht aus einem Endflansch 40 aus Stahl, der in das distale Ende des hohlen Schaftes 10 eingesetzt ist. Vorne auf den Endflansch 40 ist ein optisches Fenster 34 in Form eines Hohlkegels aus Glas, Quarzglas, Plexiglas oder Diamant aufgesetzt und z.B. durch Verkleben oder Verkitten befestigt.

Der Endflansch 40 weist die mittige axial durchgehende Bohrung auf, in welche die Optik 32 eingesetzt wird. Weiter sind in dem Endflansch 40 die vier rings um die Optik angeordnete Bohrungen für die Beleuchtungseinheiten 38 vorgesehen. Die Optik 32 und die Beleuchtungseinheiten 38 enden an der vorderen Stirnfläche des Endflansches 40. Die Beleuchtungseinheiten 38 leuchten somit den gesamten Kegel des Fensters 34 aus und die Optik 32 ermöglicht die Beobachtung des von der Spitze 12 durchdrungenen Gewebes über die gesamte Kegelfläche des Fensters 34. Die Innenfläche des Fensters 34 kann gegebenenfalls entspiegelt sein.

Zusätzlich zu oder anstelle dieser indirekten Beleuchtung kann auch eine direkte Beleuchtung bewirkt werden, wenn in die Optik 32 eine Beleuchtungseinheit z.B. in Form einer lichtführenden Lichtleiterfaser integriert ist, der ebenfalls über den Lichtleiter 18 Licht zugeführt wird.

Das Fenster 34 kann eine scharfe Spitze bilden, die ein leichtes Penetrieren des Gewebes mit dem Trokar ermöglicht. Da die Optik 32 nicht in der Stirnfläche der Spitze 12 austritt sondern in der die Basis der Spitze 12 bildenden Stirnfläche des Endflansches 40, kann auch eine Optik 32 mit größerem Durchmesser verwendet werden, z.B. ein herkömmliches Stablinsensystem (sogenannte Hopkins-Optik), die eine verbesserte Lichtausbeute aufweist. Auch hier kann die Beleuchtungseinheit 38 in die Optik 32 integriert sein. Die Optik 32 ist auch in diesem Fall selbstverständlich eine Weitwinkeloptik.

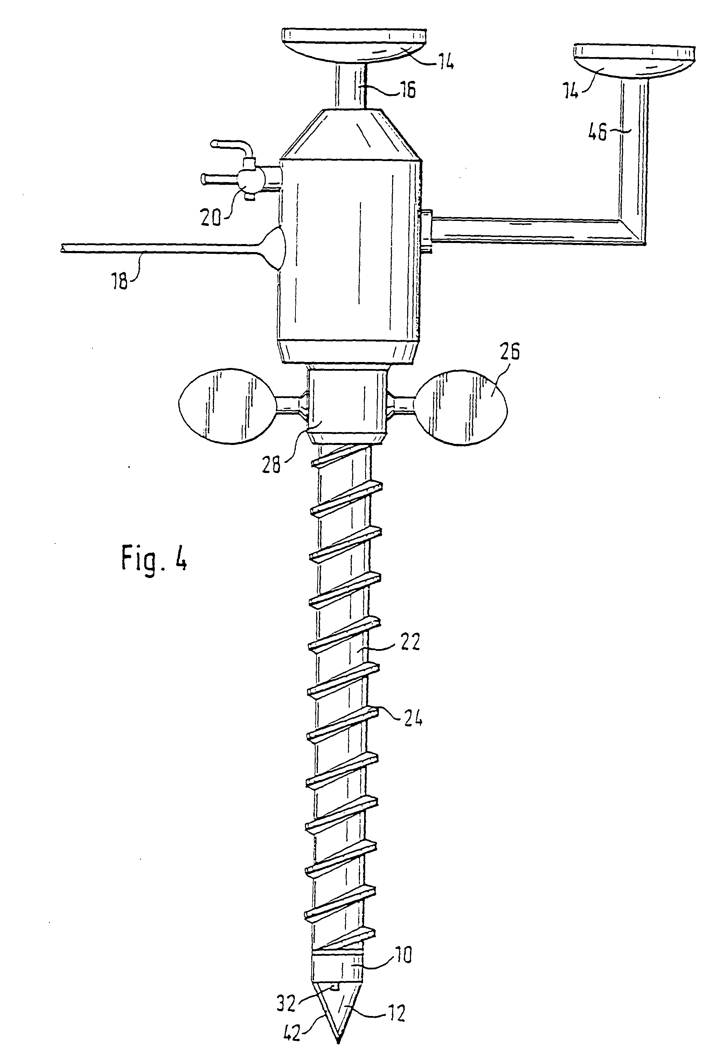

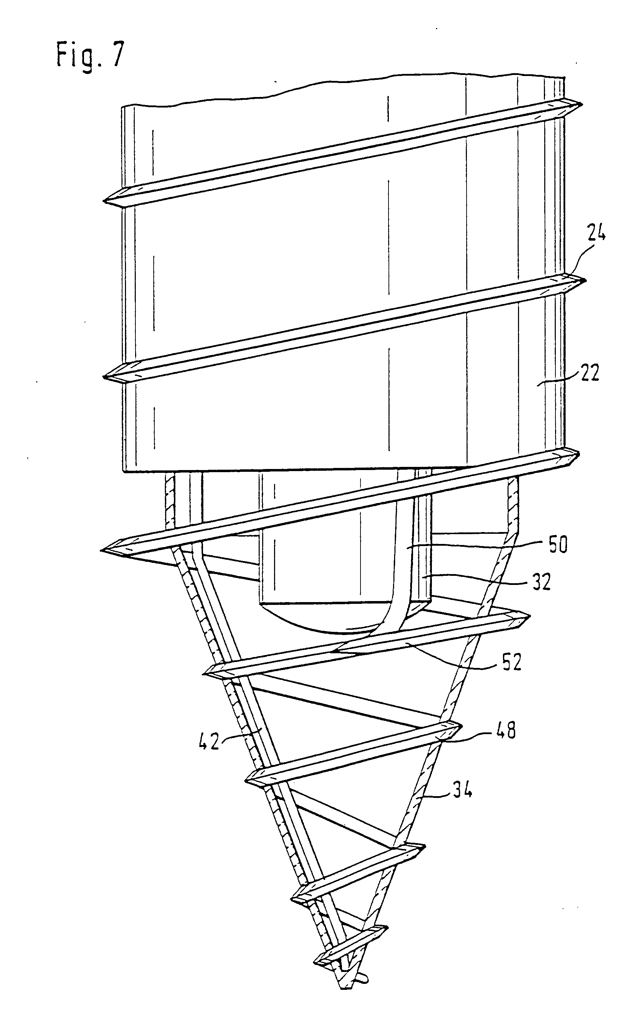

In den Figuren 4, 5 und 6 ist eine weitere Ausführungsform dargestellt, die, wie auch die der Figuren 1 und 2, nur insofern zur Erfindung gehören, wie sie das erfindungsgemäße Greifglied der Figur 7 zusätzlich aufweisen.

In dieser Ausführungsform ist das optische Fenster 34 als auf einen Endflansch 40 der Spitze 12 aufgesetzter durchsichtiger Hohlkegel ausgebildet, wie dies in Verbindung mit den Figuren 2 und 3 beschrieben ist. Axial mittig ist die erste Optik 32 eingesetzt, die vorzugsweise als Stablinsen-system die Beobachtung durch die gesamte Kegelmantelfläche des Fensters 34 erlaubt.

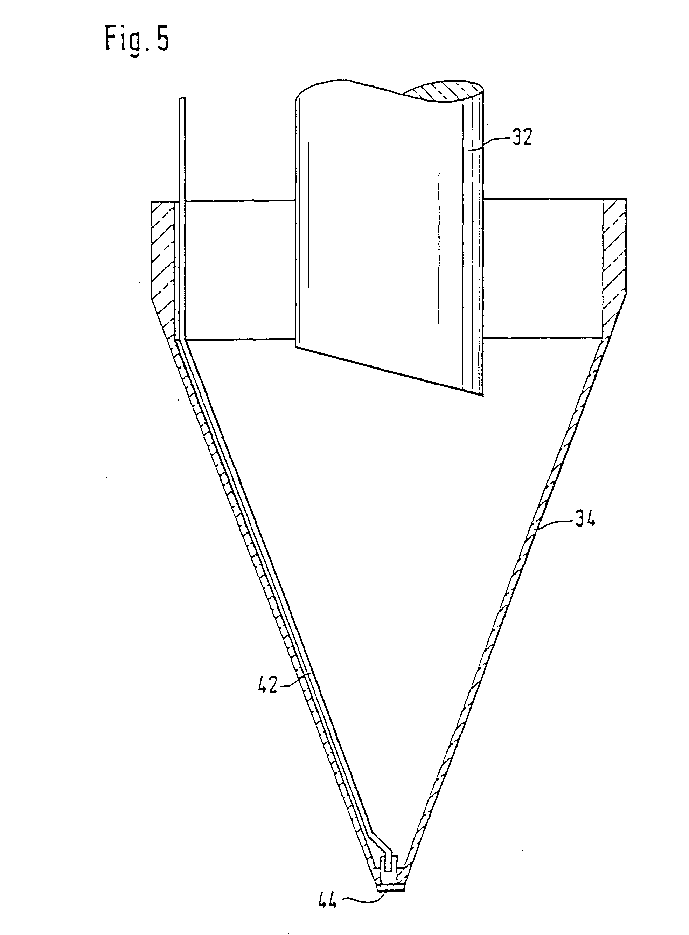

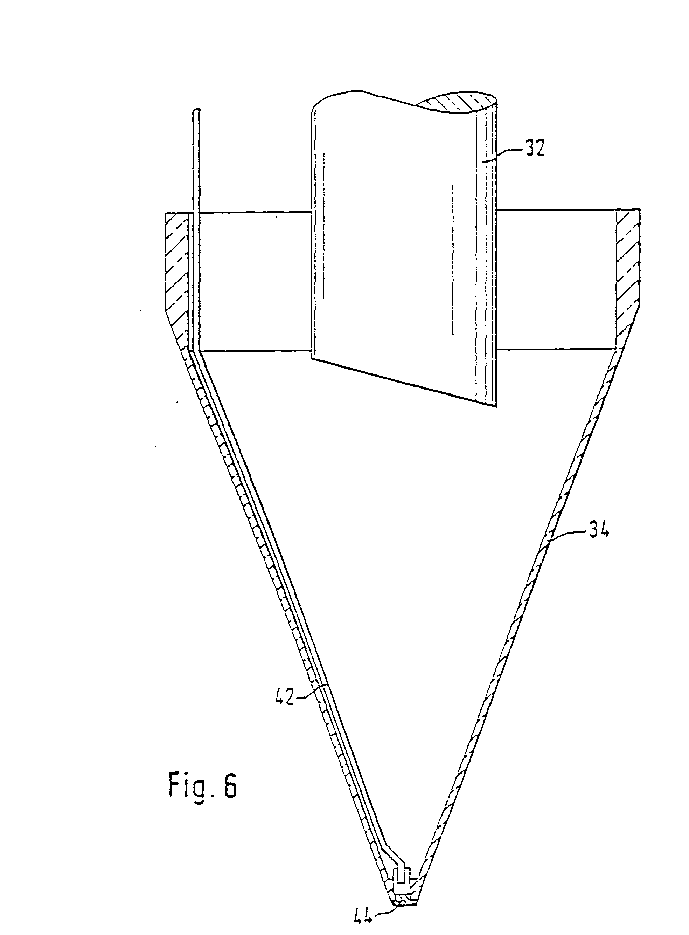

Zusätzlich zu dieser ersten Optik 32 ist eine zweite Optik 42 durch den Schart 10 zur Spitze 12 geführt. Die zweite Optik 42 ist exzentrisch zu der ersten Optik 32 durch den Endflansch 40 geführt und läuft innen an dem hohlkegeligen Fenster 34 in den Scheitelpunkt der Spitze 12. In dem Scheitelpunkt der Spitze 12 tritt die zweite Optik 42 durch die Stirnfläche des kegelförmigen Fensters 34 aus. Die Stirnfläche kann dabei durch das distal vorderste Teil der zweiten Optik 42 gebildet werden. Um die zweite Optik 42 vor Druck und Verschmutzung zu schützen, kann vor der zweiten Optik 42 ein Fenster 44 in der Stirnfläche vorgesehen sein. Dieses Fenster 44 kann als eine geschliffene Fläche aus dem Material des Fensters 34 ausgearbeitet sein, wie dies Figur 5 zeigt, oder kann in das Fenster 34 eingesetzt und z.B. durch eine Schulter axial abestützt sein, wie dies Figur 6 zeigt. Die zweite Optik 42 enthält vorzugsweise eine integrierte Beleuchtungseinheit. Die zweite Optik 42 ist vorzugsweise als dünne Lichtleiteroptik mit einem Durchmesser von 0.2 bis 0.8 mm ausgebildet. Vorzugsweise ist die zweite Optik 42 flexibel, so daß sie innen an dem Fenster 34 entlanggeführt werden kann. Das proximale hintere Ende der zweiten Optik 42 ist seitlich aus dem Schaft 10 herausgeführt und mit einem zweiten Okular 46 ausgestattet, so daß der Operateur binokular über beide Optiken 32 und 42 beobachten kann.

Die innerhalb des kegelförmigen Fensters 34 in den Scheitelpunkt der Spitze 12 geführte zweite Optik 42 behindert die Sicht der ersten Optik 32 praktisch nicht, da die zweite Optik 42 einen geringen Durchmesser hat und vorzugsweise aus durchsichtigen Glasfasern besteht.

Die Ausführungsform der Figuren 4 bis 6 bietet dem Operateur beim Einstechen des Trokars eine optimale Information. Über die erste Optik 32 und das kegelförmige Fenster 34 kann er beim Penetrieren des Gewebes die jeweils durchfahrenen Gewebsstrukturen beobachten, um die erforderlichen Informationen über die Position der Spitze und die Geschwindigkeit des Vorschubes zu erhalten. Über die zweite Optik 42 hat er die Sicht auf die vor der Spitze 12 liegenden Gewebsstrukturen unmittelbar vor deren Penetrierung. Insbesondere kann das Durchdringen des Peritoneums unter Sicht erfolgen, wobei das noch intakte semitransparente Peritoneum unmittelbar vor der Spitze 12 über die zweite Optik einen Einblick in die Bauchhöhle zuläßt, so daß beim Durchdringen des Peritoneums eine Verletzung von Gefäßen des Peritoneums und des darunterliegenden Omentum Majus und der Därme vermieden werden kann.

In der in Figur 7 dargestellten erfindungsgemäßen Ausführungsform ist an der Spitze des Trokars zusätzlich ein Greifglied angeordnet, welches dazu dient, das Körpergewebe beim Penetrieren gegen den Druck der Trokarspitze festzuhalten. Das Körpergewebe, z.B. das Peritoneum, kann dadurch nicht der Trokarspitze ausweichen und nach innen in die Abdominalhöhle eingewölbt werden.

Wie Figur 7 zeigt, besteht das Greifglied aus einer drehbar außen auf dem kegelförmigen Fenster 34 gelagerten Wendel 48, die z.B. aus einem Draht gefertigt ist und der Kegelform des Fensters 34 angepaßt ist. Die Wendel 48 ist an ihrem hinteren Ende in ein achsparalleles Verbindungsstück 50 abgebogen, welches an seinem Ende zu einem in der Zeichnung nicht sichtbaren Ring geformt ist, der drehbar in einer Umfangsnut des Schaftes 10 des Trokars gelagert ist. Mittels dieses Ringes ist die Wendel 48 somit auf dem kegelförmigen Fenster 34 drehbar gelagert und axial festgehalten. Das Schraubengewinde 24 der Hülse 22 ist über das vordere distale Hülsenende hinausgeführt und greift als Mitnehmerspitze 52 an dem hinteren Ende der Wendel 48 an, um diese bei der angetriebenen Drehung der Hülse 22 mitzunehmen.

Zum Penetrieren des Körpergewebes, z.B. der Bauchdecke des Patienten, wird die Hülse 22 drehend angetrieben, wobei sie über die Mitnehmerspitze 52 auch die Wendel 48 auf dem kegelförmigen Fenster 34 in Drehung versetzt. Durch die sich drehende Wendel 48 und das sich an diese anschließende Schraubengewinde 24 bohrt sich der Trokar nach Art eines Korkenziehers in das Gewebe, ohne daß dieses der eindringenden Trokarspitze ausweichen kann. Sobald das Gewebe penetriert ist und sich die Trokarspitze z.B. in der Abdominalhöhle befindet, kann der Trokar zusammen mit der axial auf seiner Spitze gehaltenen Wendel 48 aus der Hülse 22 herausgezogen werden. Die Hülse 22 kann dann zum Einführen von chirurgischen Instrumenten oder dergleichen verwendet werden.

## Patentansprüche

1. Instrument zum Penetrieren von Körpergewebe, mit einem hohlen Schaft (10), mit einer am distalen Ende des Schaftes (10) angeordneten Spitze (12), die zumindest in ihrem distalen Endteil als kegelförmiges Fenster (34) ausgebildet ist, mit einer Optik (32) und einer Beleuchtungseinheit (38), die in den Schaft (10) zur Spitze (12) führend eingesetzt sind, wobei das distale Ende der Optik (32) im wesentlichen symmetrisch zur Achse des Schaftes (10) und mittig zur Symmetrieachse des Fensters (34) in axialem Abstand hinter dem distalen Ende des Fensters (34) angeordnet ist, wobei das als Fenster (34) ausgebildete distale Ende der Spitze (12) dazu dient, das Gewebe zu penetrieren und die Gewebeöffnung aufzuweiten und wobei die Optik (32) zur Beobachtung des vor dem distalen Ende des Fensters (34) und seitlich an der gesamten Mantelfläche (34) des Fensters (34) liegenden Gewebes dient,
dadurch gekennzeichet daß außen auf dem Fenster (34) ein Greifglied (48) angeordnet ist, mittels dessen die Spitze (12) in das Gewebe gebohrt werden kann.

2. Instrument nach Anspruch 1,
**dadurch gekennzeichnet,** dass das Fenster (34) die Form eines Kegels oder Kegelstumpfes mit gerader, ballig gewölbter oder eingezogener Mantellinie aufweist.

## Claims

1. Instrument for penetrating bodily tissue, having a hollow shank (10), a tip (12) arranged at the distal end of the shank (10), which tip (12) is constructed at least in its distal end portion as a conical window (34), an optical system (32) and an illumination unit (38), which are inserted into the shank (10) and extend to the tip (12), the distal end of the optical system (32) being arranged substantially symmetrically relative to the axis of the shank (10) and in line with the axis of symmetry of the window (34) at an axial distance behind the distal end of the window (34), the distal end of the tip (12) constructed as a window (34) serving to penetrate the tissue and to enlarge the opening in the tissue and the optical system (32) assisting in observation of the tissue lying in front of the distal end of the window (34) and laterally over the entire circumferential surface (34) of the window (34),
characterised in that a gripping member (48) is arranged externally on the window (34), by means of which gripping member (48) the tip (12) may be bored into the tissue.

2. Instrument according to claim 1,
characterised in that the window (34) takes the form of a cone or truncated cone with straight, convexly curved or concave generatrix.

## Revendications

1. Instrument de pénétration dans des tissus corporels, dans lequel :
- une tige creuse (10) porte, à son extrémité distale une pointe (12) qui, au moins à son extrémité distale, a la forme d'une fenêtre conique (34),
- une optique (32) et une unité d'éclairage (38) sont introduites dans la tige (10) avec guidage jusqu'à la pointe (12), l'extrémité distale de l'optique (32) étant essentiellement symétrique par rapport à l'axe de la tige (10) et disposée en face de l'axe de symétrie de la fenêtre (34) à une certaine distance axiale de l'extrémité distale de la fenêtre (34),
- l'extrémité distale de la pointe (12) constituée en fenêtre (34) sert à pénétrer et à élargir l'ouverture dans le tissu, et
- l'optique (3) sert à observer le tissu se trouvant -devant l'extrémité distale de la fenêtre (34) et latéralement sur toute la surface enveloppe de la fenêtre (34),
caractérisé en ce qu'
à l'extérieur, sur la fenêtre (34) est monté un organe de prise (48) à l'aide duquel la pointe (12) peut être enfoncée par forage dans le tissu.

2. Instrument selon la revendication 1,
caractérisé en ce que
la fenêtre (34) a la forme d'un cône ou d'un tronc de cône à génératrices droites, bombées ou rentrées.
